# EUROPÄISCHE PATENTSCHRIFT

(11) **EP 3 876 837 B1**
(45) Veröffentlichungstag und Bekanntmachung des Hinweises auf die Patenterteilung: **08.10.2025**
(21) Anmeldenummer: 20710589.1
(22) Anmeldetag: 13.02.2020
(51) Int. Cl.: A61B 5/053, A61B 5/11, A61B 5/00, A61B 5/20

(54) **VORRICHTUNG FÜR DAS INTRAOPERATIVE NEUROMONITORING VON NERVEN IM BECKENBEREICH EINES PATIENTEN**
APPARATUS FOR INTRAOPERATIVE NEUROMONITORING OF NERVES IN THE PELVIC REGION OF A PATIENT
DISPOSITIF DE NEUROMONITORING PER-OPÉRATOIRE DE NERFS DANS LA ZONE DU BASSIN D'UN PATIENT

(30) Priorität: 27.02.2019 DE 102019104947
(43) Veröffentlichungstag der Anmeldung: 15.09.2021
(73) Patentinhaber: Dr. Langer Medical GmbH, 79183 Waldkirch (DE)
(72) Erfinder: LANGER, Andreas, 79183 Waldkirch (DE); SCHULER, Ramona, 79274 St. Märgen (DE)
(74) Vertreter: Maiwald GmbH
(86) Internationale Anmeldenummer: PCT/IB2020/051196
(87) Internationale Veröffentlichungsnummer: WO 2020/174304

(56) Entgegenhaltungen:
- EP-A1- 2 589 410
- WO-A1-2016/190763
- US-A1- 2007 100 387
- US-A1- 2013 079 841
- US-A1- 2015 196 220

## Beschreibung

Die Erfindung betrifft eine Vorrichtung für das intraoperative Neuromonitoring von Nerven im Beckenbereich eines Patienten, mit mindestens einem während einer Operation in das Operationsgebiet einbringbaren Nervenstimulator und mindestens einer an einem Beckenorgan des Patienten anschließbaren Sensoreinrichtung.

Im Bereich des menschlichen Beckens verläuft ein sehr filigranes Nervengeflecht, das motorische, sensorische und autonome Nervenfasern enthält. Das Risiko einer Schädigung dieses Nervengeflechts ist bei Operationen im kleinen Becken (z.B. am Rektum, der Harnblase, der Prostata oder der Gebärmutter) sehr groß und bringt stark einschränkende Folgen für die Lebensqualität der betroffenen Patienten mit sich.

Die Folgen von Nervenschädigungen des Beckens sind postoperativ auftretende Funktionsstörungen der Beckenorgane. Bis zu 38 % der Patienten leiden nach einer Operation im Bereich des Beckens an Harninkontinenz, ca. 46 % der Patienten an Stuhlinkontinenz und 18-76 % der Patienten an sexuellen Funktionsstörungen. Diese deutlichen Einschränkungen der Lebensqualität betreffen angesichts der hohen Anzahl an Operationen pro Jahr (laut Statistischem Bundesamt 2015 ca. 437200 Operationen an Rektum, Harnblase, Harnröhre und inneren Geschlechtsorganen) eine bedeutende Anzahl an Patienten.

Das Nervengeflecht des Beckens ist hochkomplex, seine Ausprägung ist äußerst fein und die Lage der vielen verschiedenen Nerven ist interindividuell verschieden. Die etablierten Methoden des Neuromonitorings, wie sie z.B. aus der Schilddrüsenchirurgie bekannt sind, lassen sich nicht ohne weiteres auf die Anwendung im Beckenbereich übertragen. Insbesondere bei Rektum-Operationen bemüht man sich schon seit einigen Jahren, nervenschonende Operationstechniken anzuwenden, jedoch hat sich noch kein System zur standardisierten und routinemäßigen intraoperativen Nervenüberwachung im Beckenbereich flächendeckend etabliert. Für den Chirurgen ist die Unterscheidung der Nerven von umliegendem Gewebe und damit die Schonung der Beckennerven sehr schwierig und erfordert fundierte Kenntnisse in der Neuroanatomie und Neurophysiologie. Die von Patient zu Patient unterschiedliche Ausprägung von Hüllfaszien und Fettgewebe im Beckenraum sowie ggf. eine Tumorinfiltration verhindert zusätzlich die Identifikation der Beckennerven.

Aus der EP 2 589 410 B1 ist eine gattungsgemäße Vorrichtung zur im Wesentlichen gleichzeitigen Kontrolle der Stimulierbarkeit von durch das vegetative Nervensystem gesteuerten Funktionen im Beckenbereich bekannt, die mindestens eine Stimulationselektrode zum intraoperativen Einbringen in den Beckenraum während einer Operation aufweist und bei der die Sensoreinrichtung als Sensoren mindestens einen zur Messung des Blasendrucks und mindestens einen zur Messung der Aktivität des Sphincter Ani internus aufweist, wobei der Sensor zur Messung der Aktivität des Sphincter ein solcher zur EMG-Messung ist. Die Verwendung eines Drucksensors zur Feststellung eines Antwortsignals bei Enervierung der Blase mittels der Stimulationselektrode hat sich als nachteilig erwiesen, denn diese Methode setzt eine zuimindest teilweise Füllung der Blase mit Flüssigkeit voraus, was während der Operation an sich unerwünscht ist. Der Einsatz eines Drucksensors an/in der Blase verlängert somit im Allgemeinen die Operationszeit in nicht unerheblichem Maße, denn es muss vor der Überprüfung der Unversehrtheit des Nervengeflechts zunächst eine Menge Flüssigkeit (Ringerlösung o.dgl.) über einen vor der Operation gelegten Blasenkatheter in die Blase eingebracht und nach erfolgter Überprüfung des/der Nerv(en) wieder abgelassen werden. Auch die intraoperative EMG-Messung am Analsphinkter konnte sich bislang in der klinischen Routine nicht durchsetzen und etablieren, denn hierfür haben sich die dabei gewonnenen Messwerte als zu unspezifisch erwiesen, so dass es an der gewünschten Zuverlässigkeit der Methode fehlt. Hintergrund ist vor allem, dass ein großer Anteil autonomer Fasern im Nervengeflecht im Beckenbereich existiert, deren Zielmuskel ein glatter Muskel ist. Glatte Muskeln enthalten Schrittmacherzellen (Cajal-Zellen), welche die Aktivität des glatten Muskels steuern und von autonomen Fasern moduliert werden. Die Kontraktion des glatten Muskels lässt sich somit schwierig durch ein herkömmliches EMG erfassen.

Eine Vorrichtung zur Messung von Volumen, Druck oder Aktivität der Harnblase mittels Impedanzmessung ist aus der US 2013/079841 bekannt.

Aufgabe der Erfindung ist es, eine Vorrichtung der eingangs genannten Art zu schaffen, mit der es möglich ist, die Unversehrtheit/Funktionstüchtigkeit von Nerven, die auf ein oder mehrere Beckenorgan(e), also insbesondere die Blase und/oder das Rektum wirken, während einer Operation im Beckenbereich eines Patienten zuverlässig und ohne nennenswerte Verzögerung überprüft werden kann. Diese Aufgabe wird mit der Erfindung dadurch gelöst, dass die Sensoreinrichtung mindestens zwei an dem Beckenorgan applizierbare Messelektroden sowie eine an die Messelektroden angeschlossene oder anschließbare Impedanz-Messeinrichtung mit einer Impedanzänderungen an dem Beckenorgan zwischen den Messelektroden anzeigenden Anzeige aufweist. Dabei soll die Formulierung "an dem Beckenorgan applizierbar" nicht nur eine unmittelbare Applikation umfassen, sondern auch eine mittelbare, d.h. eine Applikation an einem mit dem Beckenorgan in Verbindung stehenden Körperteil wie beispielsweise an der den Ausgang aus der Harnblase bildenden Harnröhre.

Es hat sich überraschend gezeigt, dass die Stimulierung von Nerven, die mit einem Beckenorgan wie der Blase oder dem Rektum in Verbindung sind, an dem betreffenden Organgewebe zu Änderungen des komplexen elektrischen Widerstands (Impedanz) führen, die als Indikator für die Unversehrtheit eines mittels des Nervenstimulators stimulierten Nervs dienen können. Mithilfe der Erfindung ist es möglich, die auftretenden Impedanzänderungen zu messen und dem Operateur an der Anzeige anzuzeigen und hierdurch eine Rückmeldung darüber zu geben, ob der stimulierte Nerv noch uneingeschränkt funktionsfähig, also unversehrt ist oder möglicherweise bei der Operation verletzt wurde.

Bei dem Nervenstimulator handelt es sich bevorzugt um eine mit einem Stimulationsstrom beaufschlagbare, vorzugsweise bipolare Stimulationselektrode, mit der es leicht möglich ist, die autonomen und somatischen Beckennerven einer direkten, elektrischen Stimulation zu unterwerfen. Dadurch lassen sich auf einfache und zuverlässige Weise reproduzierbare, stimulationsinduzierte Kontraktionen an den Beckenorganen auslösen. Die Kontraktion der glatten Muskulatur der Harnblase und des Rektums lässt sich mittels Messung der Gewebeimpedanzänderung mit der Erfindung messtechnisch erfassen. Besonders vorteilhaft ist es bei dem mit der Erfindung durchführbaren Verfahren, dass die Messung der Impedanzänderung an der Harnblase auch dann möglich ist, wenn diese leer ist, also keine Flüssigkeit enthält. Mit der Erfindung wird also die intraoperative Lokalisation und Funktionskontrolle mit den überwachten Beckenorganen in Verbindung stehenden Beckennervengeflechts möglich, und zwar ohne dass diese - beispielsweise durch Befüllen mit Ringerlösung o.dgl. - zunächst für die Durchführung einer Überprüfungsstimulation zeitaufwendig vorbereitet werden müssen.

Die Sensoreinrichtung umfasst eine Prüfstrom-Versorgungseinrichtung mit mindestens zwei an dem Beckenorgan applizierbaren Prüfstromelektroden. Dabei kann die Anordnung zweckmäßig beispielsweise so getroffen sein, dass je eine Prüfstromelektrode und eine Mess-Elektrode in einer Elektrodeneinheit zusammengefasst sind. Mit einer derartigen Anordnung lässt sich die Messtechnik zur Impedanzmessung anhand der 4 Elektrodentechnik besonders vorteilhaft realisieren. Dabei wird als Prüfstrom bevorzugt ein bekannter Konstantstrom über die zwei Prüfstromelektroden in den Körper an dem betreffenden Beckenorgan eingeprägt. Die durch die Gewebeimpedanz entstehende Spannung über dem Messabschnitt wird über die zwei Messelektroden abgegriffen. Änderungen der Gewebeimpedanz infolge einer Stimulierung eines mit dem Organ in verbindung stehenden Nerven führen zu einer Änderung der von den Messelektroden abgegriffenen Spannung, was an der Anzeigevorrichtung sichtbar ist.

Der für die Messung am Beckenorgan (Blase, Rektum) eingeprägte Konstantstrom (im folgenden Prüfstrom genannt) sollte keine Gleichstromanteile besitzen, da es aufgrund der Flüssigkeiten im Gewebe beim Anliegen von Gleichstrom zur Elektrolyse zwischen den Elektroden kommen kann. Der eingeprägte konstante Prüfstrom wird deshalb bevorzugt mit einer bestimmten Frequenz, beispielsweise einer Frequenz von 50 kHz moduliert. Zum diesem Zweck stellt die Prüfstrom-Versorgungseinrichtung in vorteilhafter Weiterbildung der Erfindung einen an den Prüfstromelektroden anliegenden Prüfwechselstrom mit festgelegter oder einstellbarer Prüfstromfrequenz bereit.

Um während einer Operation Impedanzänderungen an verschiedenen Organen überwachen zu können, kann die Prüfstrom-Versorgungseinrichtung mehrere, insbesondere zwei Prüfstromelektrodenpaare mit je zwei Prüfstromelektroden sowie Mittel zur Erzeugung von Prüfwechselströmen mit verschiedenen Prüfstromfrequenzen an den verschiedenen Prüfstromelektrodenpaaren umfassen. Die unterschiedlichen Prüfstromelektrodenpaare können dann an verschiedenen Organen, beispielsweise einerseits der Harnblase und andererseits am Rektum des zu operierenden Patienten an jeweils geeigneten Positionen appliziert werden, um an dem einen Organ einen ersten Prüfstrom mit einer ersten Prüfstromfrequenz und an dem anderen Organ einen zweiten Prüfstrom mit einer zweiten, von der ersten Prüfstromfrequenz abweichenden Frequenz anzulegen. Auf diese Weise können die von den beiden Organen mittels Messelektroden abgegriffenen Spannungen dem betreffenden Organ eindeutig zugeordnet werden.

Die Sensoreinrichtung weist vorzugsweise einen das mittels der Mess-Elektroden abgreifbare Messsignal aufbearbeitenden Messsignalverstärker, vorzugsweise einen Lock-In-Verstärker, besonders bevorzugt einen Dual-Phase Lock-In-Verstärker auf.

Als ganz besonders vorteilhaft hat es sich erwiesen, wenn die Impedanz-Messeinrichtung im Wesentlichen aus einem Impedanzspektroskop besteht. Impedanzen bestehen aus einem Realteil und einem Imaginäranteil, wobei der Realteil einem rein ohmschen Widerstand entspricht. Der Imaginärteil wird durch kapazitive und induktive Anteile bestimmt und ist frequenzabhängig. Bei der Kontraktion des glatten Muskels der überwachten Beckenorgane kann es nicht nur zu Änderungen des ohmschen Widerstands zwischen den Messelektroden, sondern auch zu Veränderungen des kapazitiven Anteils der Impedanz kommen, die sich durch die Verwendung eines Impedanzspektroskops besonders deutlich feststellen lässt. Bei der Impedanzspektroskopie wird die Modulationsfrequenz des Prüfstroms über ein bestimmtes, definiertes Frequenzspektrum variiert. Dabei lässt sich die Gewebeimpedanz in Abhängigkeit der Modulationsfrequenz ermitteln. Impedanzänderungen lassen sich dadurch sehr zuverlässig und deutlich erkennbar ermitteln.

Für eine besonders einfache und zuverlässige Applikation der Elektroden am zu überwachenden Beckenorgan hat es sich als vorteilhaft herausgestellt, wenn mindestens eine je eine Mess-Elektrode und eine Prüfstromelektrode umfassende Elektrodeneinheit in Form eines Elektrodenkatheters und/oder einer Rektalsonde ausgestaltet ist. Ein erfindungsgemäß ausgestalteter Elektrodenkatheter kann durch die Harnröhre so eingeführt werden, dass die beiden an ihm vorgesehenen Elektroden in über die Dauer der Operation zuverlässig unmittelbaren Kontakt mit dem Blasenschließmuskel bleiben. Entsprechend legen sich die zur Überwachung des Rektums an bestimmten Elektroden einer erfindungsgemäß ausgestalteten Rektalsonde am Anal Sphincter an, wenn die Sonde bestimmungsgemäß Patienten eingeführt wurde. Bei den jeweils anderen Mess- und Prüfstromelektroden kann es sich um intramural applizierbare Nadelelektroden handeln, die beispielsweise als Nadelpaar an einem gemeinsamen Nadelelektrodenhalter angeordnet sein können. Mit einem solchen Halter können sie dann vom Operateur an geeigneter, relativ zum Elektodenpaar an dem Katheter bzw. der Rektalsonde entfernter Stelle an dem jeweiligen Beckenorgan eingestochen werden.

Wie bereits vorstehend angedeutet, weist die Sensoreinrichtung vorzugsweise mindestens je zwei Mess-Elektroden und je zwei Prüfelektroden zur Applikation an der Blase und am Rektum des Patienten auf.

Weitere Merkmale und Vorteile der Erfindung ergeben sich aus der nachfolgenden Beschreibung und der Zeichnung, worin eine bevorzugte Ausführungsform der Erfindung anhand eines Beispiels näher dargestellt ist. Es zeigt:
- Fig.1: eine erfindungsgemäße Vorrichtung in einer schematischen Übersicht; und
- Fig.2: eine beispielhafte, grafische Darstellung des auf eine Stimulation eines Beckennervs bei der Impedanzmessung an einem Beckenorgan erhaltenen Antwortsignals.

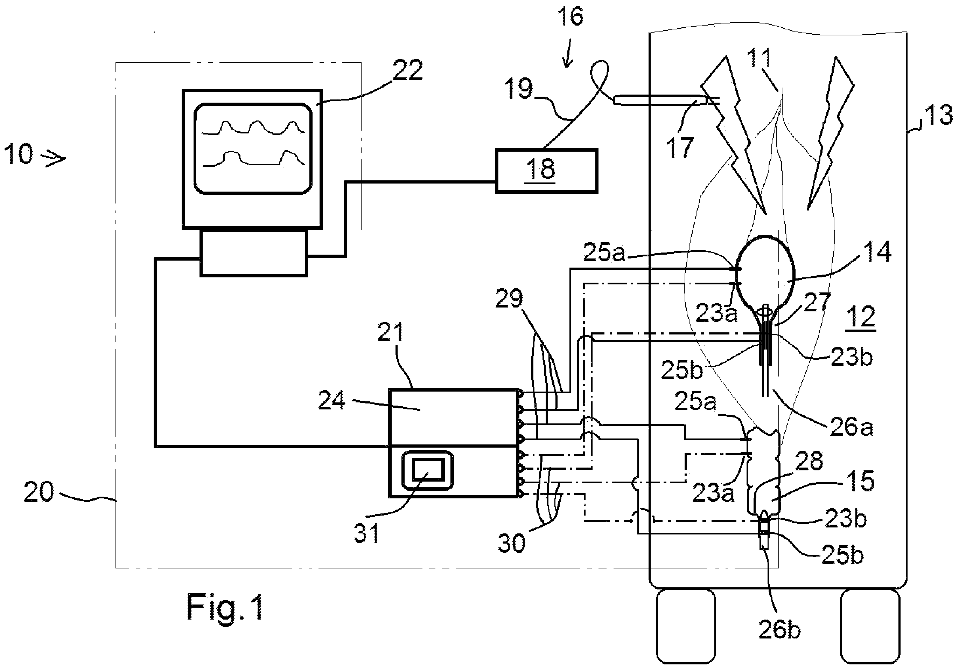

In der Fig.1 ist eine in ihrer Gesamtheit mit 10 bezeichnete Vorrichtung für das intraoperative Neuromonitoring von Nerven im Beckenbereich eines Patienten schematisch dargestellt. Die Vorrichtung 10 dient dazu, Nerven 11, die im Beckenbereich 12 eines Patienten 13 liegen und die auf eines der Beckenorgane wirken, nämlich insbesondere die Harnblase 14 und das Rektum 15, während einer Operation zu lokalisieren und/oder zu überwachen, also auf ihre Unversehrtheit und/oder Funktionalität zu überprüfen.

Für die Durchführung des intraoperativen Neuromonitoring hat die Vorrichtung 10 einen Nervenstimulator 16 in Form eines Stimulationsstromgenerators 18 und einer vom Operateur handgeführten, bipolaren Stimulationselektrode 17. Die Stimulationselektrode 17 ist mit einem Stimulationsstrom beaufschlagbar, der ihr von dem Stimulationsstromgenerator 18 über eine Leitung 19 zugeführt wird. Die Vorrichtung 10 verfügt ferner über eine in ihrer Gesamtheit mit 20 bezeichnete Sensoreinrichtung, die als Hauptbestandteile eine Impedanz-Messeinrichtung 21 mit einer Anzeige 22 sowie mehrere an den beiden Beckenorganen Harnblase 14 und Rektum 15 angeschlossene bzw. anschließbare Mess-Elektroden 23 aufweist. Die Sensoreinrichtung 20 umfasst ferner eine Prüfstrom-Versorgungseinrichtung 24 mit an den beiden Beckenorganen Harnblase 14 und Rektum 15 applizierbaren Prüfstromelektroden 25.

Die erfindungsgemäße Ausgestaltung ist so, dass für jedes der beiden Beckenorgane je zwei Prüfstromelektroden 25a, 25b und je zwei Mess-Elektroden 23a, 23b vorgesehen sind. Dabei ist die Anordnung vorliegend so getroffen, dass je eine Prüfstromelektrode 25a (bzw. 25b) und eine Meß-Elektrode 23a (bzw. 23b) in einer Elektrodeneinheit zusammengefasst sind. Eine der insgesamt vier Elektrodeneinheiten ist in Form eines Elektrodenkatheters 26a ausgestaltet, der zur Applikation an der Harnblase 14 in die Harnröhre 27 des Patienten einführbar ist, so dass sich die am Außenumfang des Katheters befindlichen Elektroden innen an die Harnröhre anlegen und so einen elektrischen Kontakt mit der Harnblase herstellen. Eine andere der Elektrodeneinheiten ist als Rektalsonde 26b ausgeführt, die entsprechend in den Anus 28 des Patienten eingeführt wird. Bei den beiden anderen Elektrodeneinheiten sind die Prüfstrom- und Mess-Elektroden als Nadelelektroden ausgeführt, die jeweils im Abstand von dem Elektrodenkatheter 26a bzw. der Rektalsonde 26b intramural an den beiden Beckenorganen 14,15 appliziert werden können.

Die Prüfstromelektroden 25 sind über Prüfstromleitungen 29 mit der Prüfstromversorgungseinrichtung 24 verbunden. In entsprechender Weise gibt es Verbindungsleitungen 30 von den Mess-Elektroden 23 hin zur Impedanz-Messeinrichtung 22, der ein integrierter Messsignalverstärker 31 in Form eines herkömmlichen Dual-Phase Lock-In-Verstärkers zugeordnet ist, mit dem die von den Mess-Elektroden 23 abgegriffenen Signale verstärkt und für eine aussagekräftige Anzeige an dem Bildschirm 22 aufbereitet werden. Die Art, wie Messsignale mittels eines Lock-In-Verstärkers aufbereitet werden können, ist dem Fachmann bekannt und soll hier nicht weiter vertieft werden.

Bei der Impedanz-Messeinrichtung handelt es sich bei der bevorzugten Ausführungsform der Erfindung um ein Impedanzspektroskop, mit dem Änderungen des Wechselstromwiderstands (Impedanz) in den Beckenorganen 14, 15 zwischen den Prüfstromelektroden 25 und den Mess-Elektroden 23 festgestellt und an der Anzeige 22 dem Operateur visualisiert werden können, die infolge einer Stimulation des/der überwachten Nerven 11 mittels der Stimulationselektrode 17 und einer daraufhin folgenden Kontraktion des Muskelgewebes des betreffenden Beckenorgans auftreten.

Hierfür ist die Vorgehensweise wie folgt:
Es wird mittels der Prüfstrom-Versorgungseinrichtung 24 ein bekannter und konstanter Prüfstrom (Wechselstrom) über die beiden jeweils am Beckenorgan 14 oder 15 applizierten Prüfstromelektroden 25a, 25b in das glatte Muskelgewebe der Harnblase bzw. des Rektums eingeprägt. Die durch die Gewebeimpedanz entstehende Spannung über dem Messabschnitt wird über die beiden Mess-Elektroden 23a, 23b abgegriffen. Dabei soll der eingeprägte Prüfstrom keine Gleichstromanteile besitzen, da es aufgrund der Flüssigkeiten im Gewebe beim Anlegen von Gleichstrom zu einer Elektrolyse zwischen den Elektroden kommen kann.

Der eingeprägte konstante Prüfstrom kann bevorzugt mit einer bestimmten Frequenz (50 kHz) moduliert werden. Da die Gewebeimpedanz nicht rein ohmsch, sondern komplex ist, entsteht aufgrund des imaginären Impedanzanteils (kapazitive und induktive Anteile) im Gewebe eine Phasenverschiebung zwischen dem Prüfstrom und der abgegriffenen Spannung über dem Messabschnitt. Die Impedanzermittlung erfolgt deshalb bevorzugt mit Bildung des Effektivwerts/ des Quadratischen Mittels (Root Mean Square-Bildung).

Der bei der in der beschriebenen Weise festgestellten Impedanz des Organgewebes über die Messstrecke enthaltene Realteil entspricht einemrein ohmschen Widerstand, während der Imaginarteil durch die kapazitiven und induktiven Anteile bestimmt wird und frequenzabhängig ist. Veränderungen der Impedanz lassen sich beispielsweise mit Hilfe der Impedanzspektroskopie deutlich feststellen. Bei der Impedanzspektroskopie wird die Modulationsfrequenz des Prüfstroms über ein bestimmtes, definiertes Frequenzspektrum variiert. Dabei lässt sich die Gewebeimpedanz in Abhängigkeit der Modulationsfrequenz ermitteln. Impedanzänderungen lassen sich dadurch besonders zuverlässig feststellen.

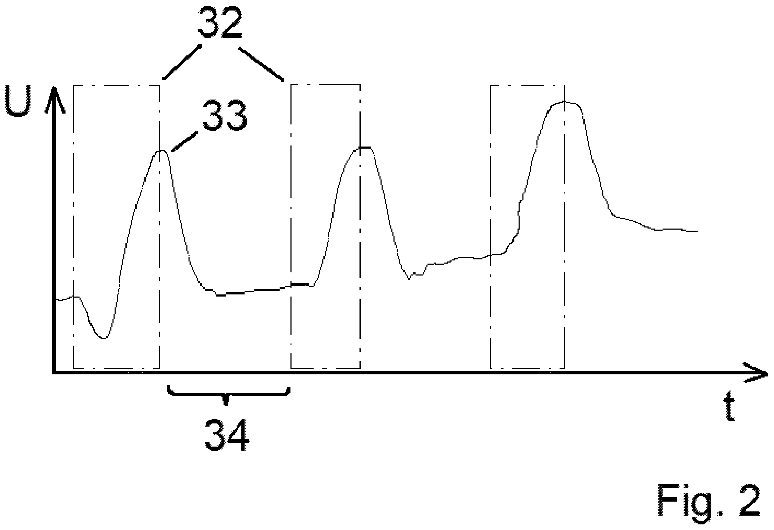

Das Ergebnis einer Impedanzmessung mit der erfindungsgemäßen Vorrichtung gemäß Fig.1 mit Hilfe der jeweils vier Elektroden an einem der beiden Beckenorgane, in Reaktion auf mehrere, im zeitlichen Abstand mittels der Stimulationselektrode 17 in einen zu überwachenden Nerv 11 eingeleitete Stimulationsstromimpulsphasen ist in der Fig.2 grafisch dargestellt. Infolge einer Stimulationsstromimpulsphase 32, bei der es sich zweckmäßig um eine Abfolge von Rechteckimpulsen mit einer festgelegten Pulsdauer eines Impulses von beispielsweise 1 ms und einer definierten Frequenz der Abfolge von z.B. 30 Hz handelt, kommt es an dem auf Unversehrtheit/Funktionsfähigkeit überwachten Nerv 11 zu einer Weiterleitung des Signals zum glatten Muskelgewebe des betroffenen Beckenorgans 14 und/oder 15, mit der Folge einer stimulationsinduzierten Muskelkontraktion und einer damit einhergehenden Veränderung der Impedanz über die Messtrecke zwischen den an dem Organ applizierten Mess-Elektroden 23a, 23b. Dementsprechend verändert sich die zwischen den Mess-Elektroden abgegriffene Spannung mit kurzer, zeitlicher Verzögerung gegenüber dem Zeitpunkt der Stimulationsstromimpulsphase, was dem Operateur an dem Bildschirm 22 angezeigt wird, beispielweise in einer Darstellung gemäß Fig.2, in der die Impedanzänderung bzw. der diese anzeigende Spannungsanstieg als Ausschlag 33 im Spannungsverlauf gut erkennbar ist. Die erneute Stimulation des Nervs 11 ist bereits unter Einhaltung einer Ruhephase 34 von weniger als 120 Sekunden möglich, in der es zu einem Abklingen der Kontraktion des glatten Muskels und damit wiederum zu einem Anstieg der Impedanz über die Messstrecke kommt.

Mithilfe der erfindungsgemäßen Vorrichtung ist es also in zuverlässiger Weise möglich, Nerven im Beckenbereich eines Patienten, insbesondere im kleinen Becken, auf ihre Unversehrtheit bzw. Funktionsfähigkeit zu überprüfen, denn ein Ausbleiben einer Impedanzänderung am betreffenden Beckenorgan trotz eines ausgelösten Stimulationsstromimpulses ist ein Hinweis darauf, dass die Nervenbahnen zwischen der Stimulationsstelle am Nerv und dem beachteten Beckenorgan beschädigt oder ganz unterbrochen sind, beispielsweise, weil sie mit einem Skalpell durchtrennt wurden. Der Operateur kann mit Hilfe der Vorrichtung in dem häufig recht schwer zu überblickenden Operationsbereich Nerven auch erst lokalisieren, um entscheiden zu können, ob ein geplanter Schnitt ohne eine Gefahr einer Beschädigung des Nervs ausgeführt werden kann.

## Patentansprüche

1. Intraoperativ-Neuromonitoring-Vorrichtung für die Lokalisierung von Nerven im Beckenbereich eines Patienten und/oder Überwachung von deren Unversehrtheit/Funktionsfähigkeit während einer Operation, mit:
- einer an mindestens einem Beckenorgan (14;15) des Patienten (13) anschließbaren Sensoreinrichtung (20), die mindestens zwei an dem Beckenorgan (14;15) applizierbare Mess-Elektroden (23) sowie eine an die Mess-Elektroden angeschlossene oder anschließbare Impedanz-Messeinrichtung (21) umfasst,
- einer Anzeige (22) zum Anzeigen von Impedanzänderungen an dem Beckenorgan (14;15) zwischen den Mess-Elektroden (23), und mit
- einer Prüfstrom-Versorgungseinrichtung (24) mit mindestens zwei an dem Beckenorgan (14;15) applizierbaren Prüfstromelektroden (25), mindestens einem während der Operation in das Operationsgebiet einbringbaren Nervenstimulator (16) in Form einer mit einem Stimulationsstrom beaufschlagbaren Stimulationselektrode (17) wobei die Vorrichtung eingerichtet ist zur Einleitung von mehreren zeitlich beabstandeten Stimulationsstromimpulsen in einen zu überwachenden Nerv (11) mittels der Stimulationselektrode (17), **dadurch gekennzeichnet, dass** die Vorrichtung so konfiguriert ist, dass sie die Impedanzänderungen zwischen den Messelektroden (23; 23b) am Beckenorgan (14; 15) in Abhängigkeit von der Zeit misst, welche Impedanzänderungen sich infolge der Erregung durch die Stimulationselektrode (17) mit den Stimulationsstromimpulsen ergeben, und die Impedanzänderungen in Abhängigkeit von der Zeit an der Anzeige so anzeigt, dass eine erste Impedanzcharakteristik zu einem ersten Zeitpunkt neben einer zweiten Impedanzcharakteristik zu einem zweiten Zeitpunkt sichtbar ist, wobei der erste Zeitpunkt von dem zweiten Zeitpunkt verschieden ist.

2. Vorrichtung nach Anspruch 1, **dadurch gekennzeichnet, dass** je eine der Prüfstromelektroden (25a;25b) und eine der Mess-Elektroden (23a;23b) in einer Elektrodeneinheit (26a;26b) zusammengefasst sind.

3. Vorrichtung nach Anspruch 1 oder 2, **dadurch gekennzeichnet, dass** die Prüfstrom-Versorgungseinrichtung (24) einen an den Prüfstromelektroden (25) anliegenden Prüfwechselstrom mit festgelegter oder einstellbarer Prüfstromfrequenz bereitstellt.

4. Vorrichtung nach einem der Ansprüche 1 bis 3, **dadurch gekennzeichnet, dass** die Prüfstrom-Versorgungseinrichtung (24) mehrere, insbesondere zwei Prüfstromelektrodenpaare mit je zwei Prüfstromelektroden (25a,25b) sowie Mittel zur Erzeugung von Prüfwechselströmen mit verschiedenen Prüfstromfrequenzen an den verschiedenen Prüfstromelektrodenpaaren umfasst.

5. Vorrichtung nach einem der Ansprüche 1 bis 4, **dadurch gekennzeichnet, dass** die Sensoreinrichtung (20) einen das an den Mess-Elektroden (23) abgreifbare Messsignal aufbereitenden Messsignalverstärker (31), vorzugsweise einen Lock-In-Verstärker, besonders bevorzugt einen Dual-Phase Lock-In-Verstärker aufweist.

6. Vorrichtung nach einem der Ansprüche 1 bis 5, **dadurch gekennzeichnet, dass** die Impedanz-Messeinrichtung (21) im Wesentlichen aus einem Impedanzspektroskop besteht.

7. Vorrichtung nach einem der Ansprüche 2 bis 6, **dadurch gekennzeichnet, dass** mindestens eine je eine Mess-Elektrode (23a;23b) und eine Prüfstromelektrode (25a;25b) umfassende Elektrodeneinheit in Form eines Elektrodenkatheters (26a) und/oder einer Rektalsonde (26b) ausgestaltet ist.

8. Vorrichtung nach einem der Ansprüche 1 bis 7, **dadurch gekennzeichnet, dass** der Nervenstimulator (16) eine bipolare Stimulationselektrode (17) ist.

9. Vorrichtung nach einem der Ansprüche 1 bis 8, **dadurch gekennzeichnet, dass** die Sensoreinrichtung (20) mindestens je zwei Mess-Elektroden (23 a,b) und je zwei Prüfelektroden (25 a,b) zur Applikation an der Blase (14) und am Rektum (15) des Patienten aufweist.

## Claims

1. Intraoperative neuromonitoring apparatus for locating nerves in the pelvic region of a patient and/or monitoring their integrity/functionality during an operation, having:
- a sensor device (20) which can be connected to at least one pelvic organ (14; 15) of the patient (13) and comprises at least two measuring electrodes (23) which can be applied to the pelvic organ (14; 15) and an impedance measuring device (21) which is connected or can be connected to the measuring electrodes,
- a display (22) for displaying impedance changes on the pelvic organ (14; 15) between the measuring electrodes (23), and having
- a test current supply device (24) with at least two test current electrodes (25) which can be applied to the pelvic organ (14; 15),
at least one nerve stimulator (16), which can be introduced into the operating area during the operation and is in the form of a stimulation electrode (17) to which a stimulation current can be applied, wherein the apparatus is configured to introduce a plurality of temporally spaced stimulation current pulses into a nerve (11) to be monitored by means of the stimulation electrode (17), **characterized in that** the apparatus is configured such that it measures the impedance changes between the measuring electrodes (23; 23b) on the pelvic organ (14; 15) on the basis of the time, which impedance changes result from the excitation by the stimulation electrode (17) with the stimulation current pulses, and displays the impedance changes on the basis of the time on the display such that a first impedance characteristic is visible at a first time in addition to a second impedance characteristic at a second time, wherein the first time is different from the second time.

2. Apparatus according to Claim 1, **characterized in that** one of the test current electrodes (25a; 25b) and one of the measuring electrodes (23a; 23b) are combined in an electrode unit (26a; 26b).

3. Apparatus according to Claim 1 or 2, **characterized in that** the test current supply device (24) provides an alternating test current which is applied to the test current electrodes (25) and has a fixed or adjustable test current frequency.

4. Apparatus according to one of Claims 1 to 3, **characterized in that** the test current supply device (24) comprises a plurality of, in particular two, test current electrode pairs with two test current electrodes (25a, 25b) each and means for generating alternating test currents with different test current frequencies at the different test current electrode pairs.

5. Apparatus according to one of Claims 1 to 4, **characterized in that** the sensor device (20) has a measurement signal amplifier (31), preferably a lock-in amplifier, particularly preferably a dual-phase lock-in amplifier, which conditions the measurement signal that can be tapped off at the measuring electrodes (23).

6. Apparatus according to one of Claims 1 to 5, **characterized in that** the impedance measuring device (21) essentially consists of an impedance spectroscope.

7. Apparatus according to one of Claims 2 to 6, **characterized in that** at least one electrode unit comprising one measuring electrode (23a; 23b) and one test current electrode (25a; 25b) is in the form of an electrode catheter (26a) and/or a rectal probe (26b).

8. Apparatus according to one of Claims 1 to 7, **characterized in that** the nerve stimulator (16) is a bipolar stimulation electrode (17).

9. Apparatus according to one of Claims 1 to 8, **characterized in that** the sensor device (20) has at least two measuring electrodes (23 a,b) and two test electrodes (25 a,b) each for application to the bladder (14) and to the rectum (15) of the patient.

## Revendications

1. Dispositif de neuromonitoring peropératoire pour la localisation de nerfs dans la zone du bassin d'un patient et/ou pour la surveillance de leur intégrité/de leur bon fonctionnement au cours d'une intervention, avec :
- un système de capteur (20) pouvant être raccordé à au moins un organe (14 ; 15) du bassin du patient (13), qui comprend au moins deux électrodes de mesure (23) pouvant être appliquées sur l'organe (14 ; 15) du bassin ainsi qu'un système de mesure d'impédance (21) raccordé ou pouvant être raccordé aux électrodes de mesure,
- un affichage (22) destiné à afficher des variations d'impédance sur l'organe (14 ; 15) du bassin entre les électrodes de mesure (23), et avec
- un système d'alimentation en courant de test (24) avec au moins deux électrodes de courant de test (25) pouvant être appliquées sur l'organe (14 ; 15) du bassin, au moins un neurostimulateur (16) pouvant être introduit dans le champ opératoire au cours de l'intervention sous la forme d'une électrode de stimulation (17) pouvant être soumise à l'action d'un courant de stimulation, le dispositif étant mis au point pour appliquer plusieurs impulsions de courant de stimulation espacées dans le temps dans un nerf (11) à surveiller au moyen de l'électrode de stimulation (17), **caractérisé en ce que** le dispositif est configuré de telle sorte qu'il mesure en fonction du temps les variations d'impédance entre les électrodes de mesure (23 ; 23b) sur l'organe (14 ; 15) du bassin, lesquelles variations d'impédance résultent de l'excitation par l'électrode de stimulation (17) avec les impulsions de courant de stimulation, et affiche les variations d'impédance en fonction du temps sur l'affichage de telle sorte qu'une première caractéristique d'impédance à un premier moment est visible à côté d'une deuxième caractéristique d'impédance à un deuxième moment, le premier moment étant différent du deuxième moment.

2. Dispositif selon la revendication 1, **caractérisé en ce que** respectivement une des électrodes de courant de test (25a ; 25b) et une des électrodes de mesure (23a ; 23b) sont regroupées dans une unité d'électrodes (26a ; 26b).

3. Dispositif selon la revendication 1 ou 2, **caractérisé en ce que** le système d'alimentation en courant de test (24) fournit un courant alternatif de test appliqué sur les électrodes de courant de test (25) avec une fréquence de courant de test fixée ou réglable.

4. Dispositif selon l'une des revendications 1 à 3, **caractérisé en ce que** le système d'alimentation en courant de test (24) comprend plusieurs, en particulier deux, paires d'électrodes de courant de test avec respectivement deux électrodes de courant de test (25a, 25b) ainsi que des moyens de production de courants alternatifs de test avec différentes fréquences de courant de test sur les différentes paires d'électrodes de courant de test.

5. Dispositif selon l'une des revendications 1 à 4, **caractérisé en ce que** le système de capteur (20) comporte un amplificateur de signal de mesure (31) préparant le signal de mesure pouvant être prélevé sur les électrodes de mesure (23), de préférence un amplificateur synchrone, de manière particulièrement préférée un amplificateur synchrone à double phase.

6. Dispositif selon l'une des revendications 1 à 5, **caractérisé en ce que** le système de mesure d'impédance (21) est constitué sensiblement d'un spectroscope d'impédance.

7. Dispositif selon l'une des revendications 2 à 6, **caractérisé en ce qu'**au moins une unité d'électrodes comprenant respectivement une électrode de mesure (23a ; 23b) et une électrode de courant de test (25a ; 25b) est configurée sous la forme d'un cathéter à électrodes (26a) et/ou d'une sonde rectale (26b).

8. Dispositif selon l'une des revendications 1 à 7, **caractérisé en ce que** le neurostimulateur (16) est une électrode de stimulation (17) bipolaire.

9. Dispositif selon l'une des revendications 1 à 8, **caractérisé en ce que** le système de capteur (20) comporte au moins respectivement deux électrodes de mesure (23 a,b) et respectivement deux électrodes de test (25 a,b) destinées à être appliquées sur la vessie (14) ou le rectum (15) du patient.
